**Europäisches Patentamt**

**European Patent Office**

**Office européen des brevets**

(19)

(11) Veröffentlichungsnummer: **0 095 177**
**B1**

(12) # EUROPÄISCHE PATENTSCHRIFT

(45) Veröffentlichungstag der Patentschrift:
**24.07.85**

(51) Int. Cl.⁴: **C 07 C 143/675, C 07 C 143/70**

(21) Anmeldenummer: **83105085.1**

(22) Anmeldetag: **21.05.83**

(54) **Verfahren zur Herstellung von N-Acetylaminoarylsulfonsäuren.**

(30) Priorität: **26.05.82 DE 3219651**

(43) Veröffentlichungstag der Anmeldung:
**30.11.83 Patentblatt 83/48**

(45) Bekanntmachung des Hinweises auf die Patenterteilung:
**24.07.85 Patentblatt 85/30**

(84) Benannte Vertragsstaaten:
**BE CH DE FR GB IT LI**

(56) Entgegenhaltungen:
**DE - A - 2 702 584**
**DE - C - 92 796**

**ULLMANNS ENCYKLOPÄDIE DER TECHNISCHEN CHEMIE, 4. Auflage, Band 8, 1974 VERLAG CHEMIE, Weinheim/Bergstrasse**
**BEILSTEINS HANDBUCH DER ORGANISCHEN CHEMIE, 4. Auflage, Band 14, 1931 VERLAG JULIUS SPRINGER, Berlin**

(73) Patentinhaber: **HOECHST AKTIENGESELLSCHAFT, Postfach 80 03 20, D-6230 Frankfurt am Main 80 (DE)**

(72) Erfinder: **Arndt, Otto, Dr., Frankfurter Strasse 38, D-6238 Hofheim am Taunus (DE)**
Erfinder: **Papenfuhs, Theodor, Dr., Heinrich-Bleicher-Strasse 40, D-6000 Frankfurt am Main 50 (DE)**

## Beschreibung

Die Erfindung betrifft salzfreie N-Acetylaminoarylsulfonsäuren, ein Verfahren zu ihrer Herstellung und ihre Verwendung zur Herstellung der N-Acetylaminoarylsulfochloride.

Salze der N-Acetylaminosulfonsäuren, insbesondere des Benzols und des Naphthalins, sind seit langem bekannt, ebenso zum Teil auch die entsprechenden freien Säuren, z. B. die N-Acetylsulfanilsäure (p-Acetaminobenzolsulfonsäure, p-Acetanilinsulfonsäure) und die N-Acetylnaphthionsäure (4-Acetaminonaphthalinsulfonsäure-1).

Zur Herstellung dieser N-Acetylaminoarylsulfonsäuren kann grundsätzlich unterschieden werden zwischen der Einführung von Sulfonsäuregruppen in N-Acetylaminoarene einerseits und der Acetylierung an der Aminogruppe von Aminoarylsulfonsäuren.

Aus der erstgenannten Gruppe von Herstellungsverfahren seien genannt: Eintragen von Acetanilid in rauchende Schwefelsäure und mehrstündiges Erwärmen der entstandenen Lösung; eine Isolierung von N-Acetylaminobenzolsulfonsäure war nicht möglich [„Ber.", *18* (1885) 296]. Dagegen konnten mit rauchender Schwefelsäure p-Acetanilinsulfonsäure und N-acetylierte Sulfonsäuren von p-Toluidin und von 1,4,2- und 1,3,4-Xylidin in kristalliner Form erhalten werden [„Ber.", *33* (1900) 1364/6]. N-Acetylsulfanilsäu-re wurde auch aus Acetanilid mit Acetylschwefelsäure (aus konz. Schwefelsäure und Essigsäureanhydrid) erhalten [„Ber.", *42* (1909) 4539]. Aufgrund kinetischer Studien wurde festgestellt, dass bei Konzentrationen >70% von wasserhaltigen Schwefelsäuren die Sulfierung die Hydrolyse der N-Acetylgruppe überwiegt [„Chem. & Ind.", *36* (1970) 1172, „Tetrahedron Letters" (1971) 2161/2]. Schliesslich wurden zur Einführung von Sulfonsäuregruppen noch Kernhalogensubstitutionsprodukte von Acetanilid umgesetzt mit sauren bzw. neutralen Sulfiten unter Bildung von N-Acetylaminobenzolsulfonsäuren bzw. von ihren Salzen (DE-PS Nr. 101777, „Friedl.", *5*, 754/6).

Alle diese Verfahren eignen sich nicht zur Herstellung von N-Acetylaminoarylsulfonsäuren im technischen Massstab.

Aus der zweiten obengenannten Gruppe von Herstellverfahren seien genannt:

Umsetzung von z. B. sulfanilsaurem Natrium in wässeriger Lösung mit Keten (DE-PS Nr. 453577, „Friedl.", *16*, 237/9).

Dieses Verfahren eignet sich wegen der Giftigkeit und schlechten Handhabbarkeit des Ketens wenig zur Durchführung im technischen Massstab.

Umsetzung von trockenen, pulverförmigen Na-, K-, Ba-Salzen von Aminosulfonsäuren des Benzols und Naphthalins mit Essigsäureanhydrid [„Ber.", *17* (1884) 707/8, *21* (1888) 2579/82, *39* (1906) 1559/70, *58* (1925) 2287, „J. Prakt. Chem." (2) *63* (1901) 406/9], des Na-Salzes mit Eisessig (DE-PS Nr. 92796, „Friedl.", *4*, 1152).

Umsetzung von Salzen der Aminosulfonsäuren der Benzol- und Naphthalinreihe in wässeriger Lösung oder Suspension mit Essigsäureanhydrid [DE-PS Nr. 129000, „Friedl.", *6*, 215/7, „Ber.", *46* (1913) 777, „Bull. Soc. Chim. France" (5) *21* (1954) 97/8], mit Essigsäure und Essigsäureanhydrid [DE-PS Nr. 410364, „Friedl.", *15*, 133, „Ber.", *33* (1900) 417, „J. Org. Chem.", 6, 25 (1941)].

Umsetzung von Aminosulfonsäuren des Benzols und Naphthalins in Pyridin mit Essigsäureanhydrid, ggf. unter Zusatz von Eisessig [„J. Soc. Chem. Ind.", *46* (1927) 224T/226T, *47* (1928) 155T-157T, „J. Soc. Dyers Colourists", *59* (1943) 144/8, „Synthesis" 12 (1974) 877/8].

Während bei den vorgenannten Verfahren die Acetylierung in stark exothermer Reaktion zu den Salzen (Metall- bzw. Pyridiniumsalzen) der N-Acetylaminoarylsulfonsäuren führt, gelingt die Acetylierug der freien Aminoarylsulfonsäuren nur in Ausnahmefällen, z. B. bei der o-Toluidinsulfonsäure [„Ber.", *17* (1884) 707/8]. Die o-, m- und p-Aminobenzolsulfonsäuren reagieren auch beim 10-stündigen Erhitzen mit Essigsäureanhydrid auf 100 bis 140° C nicht [„Bull. Soc. Chim. France" (5) *21* (1954) 98].

Das bevorzugte technische Verfahren zur Herstellung des Na-Salzes von N-Acetylsulfanilsäure ist bisher die Umsetzung von sulfanilsaurem Na (aus Sulfanilsäure und Soda) in wässeriger Lö-sung mit Essigsäureanhydrid („Ullmanns Ency-clopädie der Technischen Chemie", 4. Aufl., Bd. 8, S. 426 nach „BIOS Final Rept.", Nr. 1149, S. 125/6).

Die freien N-Acetylaminoarylsulfonsäuren werden aus den wässerigen Lösungen ihrer Salze durch Behandeln mit starken Mineralsäuren erhalten [„DE-Anm. J", 4736, „Friedl.", *5* 756, „Ber.", *39* (1906) 1559/70]. Es fallen dabei Säuren an, die erhebliche Mengen an mineralsauren Salzen enthalten, die eine Regenerierung der Säuren zwar nicht unmöglich machen, aber die damit verbundenen Kosten stark erhöhen.

Aus Kosten- und ökologischen Gründen besteht ein dringender Bedarf an einem Verfahren, bei dem die Aminoarylsulfonsäuren in freier Form acetyliert werden können.

Es wurde nun gefunden, dass sich Aminoaryl-sulfonsäure in Schwefelsäure als Lösungsmittel mit Acetanhydrid oder Acetylchlorid zu N-Acetylaminoarylsulfonsäuren acetylieren lassen.

Gefunden wurde ein Verfahren zur Herstellung von N-Acetylaminoarylsulfonsäuren durch Acety-lierung von Aminoarylsulfonsäuren mit Essigsäu-reanhydrid oder Acetylchlorid, dadurch gekenn-zeichnet, dass man die Acetylierung in Schwefelsäure als Lösungsmittel vornimmt.

Im Falle des Essigsäureanhydrids bildet sich Essigsäure, die im Reaktionsgemisch verbleibt, im Falle des Acetylchlorids entweicht HCl gasförmig.

Die Schwefelsäure soll vorzugsweise eine geringe Menge Wasser und/oder Dimethylformamid und/oder N-Methylpyrrolidon enthalten. Zweck-mässigerweise werden bis zu 2 Gew.-%, insbesondere bis zu 1 Gew.-%, Wasser bzw. bis zu 3 Gew.-%, insbesondere bis zu 2,5 Gew.-%, Dimethylformamid oder N-Methylpyrrolidon eingesetzt, jeweils bezogen auf die Schwefelsäure.

Um das Reaktionsgemisch gut rührbar zu erhalten, wird die Schwefelsäure zweckmässigerweise in einer Mindestmenge von etwa 4 bis 5 mol je Mol Aminoarylsulfonsäure eingesetzt für den Fall, dass nur die Schwefelsäure vorgelegt wird. Wird mit der Schwefelsäure auch schon das Acetylierungsmittel Essigsäureanhydrid oder Acetylchlorid vorgelegt, dann reduziert sich die Mindest-Schwefelsäuremenge auf etwa 2 bis 3 mol je Mol Aminoarylsulfonsäure.

Das Acetylierungsmittel wird zweckmässigerweise in einer Menge von etwa 1,25 bis etwa 2 mol je Mol Aminoarylsulfonsäure eingesetzt.

Zweckmässigerweise werden Schwefelsäure bzw. Schwefelsäure und Acetylierungsmittel vorgelegt und bei Raumtemperatur (etwa 25° C) die wasser- und salzfreie, pulverförmige Aminoarylsulfonsäure und das Acetylierungsmittel bzw. nur die Aminoarylsulfonsäure eingetragen. Zuweilen ist es nötig, zur Verbesserung der Rührbarkeit des Reaktionsgemisches noch während dieses Eintragens die Temperatur etwas zu erhöhen. Nach erfolgtem Eintragen wird die Temperatur dann auf etwa 60 bis 90° C, je nach der zu acetylierenden Aminoarylsulfonsäure, erhöht.

Das Fortschreiten der Acetylierung wird zweckmässigerweise mittels Dünnschichtchromatographie der noch verbliebenen nicht acetylierten Aminosulfonsäure verfolgt. Falls erforderlich, kann noch fehlendes Acetylierungsmittel nachgeschleust werden.

Man erhält im allgemeinen gut rührbare, mässig viskose Lösungen bzw. Suspensionen der N-Acetylaminoarylsulfonsäuren, die entweder direkt zur Umsetzung zum entsprechenden Sulfonsäurechlorid verwendet werden können, oder zur Isolierung der N-Acetylaminoarylsulfonsäuren noch heiss in vorgelegtes Wasser unter Rühren abgelassen werden.

Dabei kristallisieren die Hydrate der N-Acetylaminoarylsulfonsäuren aus. Zur Erzielung einer guten Filtrierbarkeit wird zweckmässigerweise eine Temperatur von etwa 30 bis etwa 40° C eingehalten. Die vorgelegte Wassermenge wird zweckmässigerweise so abgestimmt, dass man Filtratsäuren mit einem Gehalt von mindestens etwa 30% Schwefelsäure und mindestens etwa 20% Essigsäure erhält.

Die abfiltrierten N-Acetylaminoarylsulfonsäuren werden zweckmässigerweise mit etwa 3 bis 6 mol HCl in Form von etwa 20-30%iger Salzsäure schwefelsäurefrei gewaschen. Zweckmässigerweise wird das Nutschgut in einen Teil der Salzsäure verrührt, erneut filtriert und mit dem Rest der Salzsäure nachgewaschen. Man kann jedoch auch das Produkt auf der Nutsche belassen und die gesamte Salzsäuremenge zur Wäsche benutzen.

Bei der Wäsche erhält man eine Salzsäure, die noch einige Prozent Schwefelsäure und auch Essigsäure enthält.

Wegen der guten Wasserlöslichkeit der N-Acetylaminoarylsulfonsäuren wäre eine abschliessende Wäsche mit Wasser mit mehr oder weniger grossen Substanzverlusten verbunden. Die geschilderte Salzsäurewäsche jedoch reduziert die Verluste erheblich. Man erhält die N-Acetylaminoarylsulfonsäuren als schwefelsäurefreie, salzsäurehaltige Feuchtprodukte, die von HCl und Wasser zweckmässigerweise in einem korrosionsfesten Trockner, z. B. in einem emaillierten Doppelkonustrockner bei Temperaturen bis 120° C, vorzugsweise bei reduzierten Drücken, befreit werden können. Die getrockneten Produkte enthalten höchstens Spuren Aminoarylsulfonsäure und geringe Mengen Wasser. Die so getrockneten Produkte werden ggf. noch gemahlen. Sie lassen sich dann gut dosieren.

Die Ausbeuten liegen bei 90 bis 95% d. Th.

Die Feuchtprodukte lassen sich auch durch Erhitzen mit organischen Stoffen, z. B. höher siedenden Benzinfraktionen (insbesondere mit Siedepunkten von 110 bis 140° C) und Abdestillieren dieser Schleppmittel im emaillierten Rührkessel entwässern.

Das Auswaschen der Schwefelsäure muss möglichst quantitativ erfolgen, damit bei den hohen Trocknungstemperaturen keine Sulfurierungen der erhaltenen N-Acetylaminoarylsulfonsäuren erfolgen.

Die Filtratsäuren (Schwefelsäure mit Essigsäure und Salzsäure mit geringen Mengen Schwefelsäure und Essigsäure) werden in bekannter Weise von flüchtigen Anteilen befreit (HCl und Essigsäure) und aufkonzentriert. Diese Regenerierung ist unproblematisch, da keine störenden Salze enthalten sind. Sie hinterlässt auch keine Rückstände, die kanalisiert werden müssten.

Nach dem erfindungsgemässen Verfahren lassen sich Aminoarylsulfonsäuren, vorzugsweise des Benzols und Naphthalins, die auch am Kern substituiert, vorzugsweise alkylsubstituiert, besonders bevorzugt methyliert sein können, insbesondere die o-, m- und p-Aminobenzolsulfonsäuren und die Aminonaphthalinsulfonsäuren-1,4, -2,6 und -2,8 zu ihren N-Acetyl-Derivaten acetylieren.

Die erhaltenen N-Acetylaminoarylsulfonsäuren können entweder, wie oben schon erwähnt, in Form der nach der Acetylierungsreaktion erhaltenen Lösung oder Suspension oder als isolierte, getrocknete Substanzen verwendet werden zur Herstellung der entsprechenden Sulfonsäurechloride.

Gemäss Stand der Technik sind folgende Verfahren zur Herstellung von N-Acetylaminoarylsulfochloriden beschrieben worden:

Aus Acetanilid und Chlorsulfonsäure bzw. Oleum und Chlorsulfonsäure, wobei zusätzlich noch andere chlorierend wirkende Mittel von Säurechloridcharakter, z. B. Phosphortrichlorid und Phosphorpentachlorid sowie bevorzugt Thionylchlorid, angewendet werden [DE-PS Nr. 752572, „Ber.'', *39* (1906) 1559/70, *46* (1913) 777, „Zh. Prikl. Khim.'', *11* (1938) 316/27, „C.Z.'' (1939) I 4934/6].

Umsetzung von Metallsalzen [„Zh. Prikl. Khim.'', *34* (1961) 1625/7, „J. Appl. Chem. USSR (English Translation)'', *34* (1961) 1549/51, DE-PS Nr. 532399, „Friedl.", *18* 601/4, DD-PS Nr. 30312, „Organic Syntheses Collective", Vol. I, S. 8/10, New York-London, 2. Aufl.] oder von Pyridiniumsalzen [„Synthesis"] (1974) S. 877/8] von N-Acetylaminoarylsulfonsäuren mit Chlorsulfonsäure oder mit Phosphorpentachlorid [„Ann." *380* (1911) 309].

Während die Sulfochlorierung von Acetaminoaromaten nur zu einigen wenigen Vertretern der Acetaminoarylsulfochloride führt, und dies auch in unbefriedigenden Ausbeuten, ist die Herstellung über die Salze der N-Acetylaminoarylsulfonsäuren mit den gleichen Nachteilen behaftet, wie sie oben für die Herstellung der freien N-Acetylaminoarylsulfonsäuren beschrieben sind:

Anfall von erheblichen Mengen an Filtratsäuren, deren Regenerierung durch die in ihnen enthaltenen Metallionen stark gestört wird.

Die Verwendung der erfindungsgemäss erhaltenen salzfreien N-Acetylaminoarylsulfonsäuren eröffnet eine Möglichkeit zur salzfreien Herstellung ihrer Chloride.

Bei Verwendung der nach der Acetylierungsreaktion erhaltenen Lösung oder Suspension ist ein Überschuss an Thionylchlorid als bevorzugtes Chlorierungsmittel erforderlich, der davon abhängt, in wieviel Schwefelsäure die N-Acetylaminoarylsulfonsäure gelöst ist. Die Schwefelsäure reagiert mit dem Thionylchlorid unter Bildung von Chlorsulfonsäure. Gleichzeitig wird die N-Acetylaminoarylsulfonsäure zum Säurechlorid chloriert. Ggf. kann vor der Thionylchloridzugabe Chlorsulfonsäure der Lösung der N-Acetylaminoarylsulfonsäure in Schwefelsäure zugefügt werden.

Verwendet man die isolierte, getrocknete N-Acetylaminoarylsulfonsäure, so wird diese zweckmässigerweise in Chlorsulfonsäure gelöst und mit Thionylchlorid zum Säurechlorid chloriert.

In beiden Fällen entstehen bei dieser bevorzugten Verwendung von Thionylchlorid als Chlorierungsmittel $SO_2$ und HCl als Abgase, die in bekannter Weise in Vorrichtungen zur Gaswäsche problemlos abgetrennt werden.

In beiden Fällen wird die Umsetzung mit Thionylchlorid zweckmässigerweise bei 30 bis 70° C, vorzugsweise 50 bis 60° C, vorgenommen. Nach beendeter Zugabe des Thionylchlorids wird bei unveränderter Temperatur noch einige Zeit nachgerührt.

Die Umsetzung mit Thionylchlorid wird durch die Zugabe geringer Mengen Dimethylformamid gefördert [„Helv. Chim. Acta", *42* (1959) (1653-1658)].

Nach beendeter Gasentwicklung wird das erhaltene Reaktionsgemisch in Eiswasser abgelassen. Dabei fällt das N-Acetylaminoarylsulfonsäurechlorid aus. Das gefällte Produkt wird abgesaugt, mit Eiswasser gewaschen und entweder in feuchter Form weiterverwendet oder zweckmässigerweise im Trockenschrank in dünner Schicht bei Unterdruck und einer Temperatur bis etwa 50° C und unter Durchleiten eines trockenen Stickstoffstroms getrocknet.

Wie bei der erfindungsgemässen Herstellung der N-Acetylaminoarylsulfonsäuren fallen bei der Herstellung ihrer Säurechloride Filtrat- und Waschflüssigkeiten an, die sich problemlos regenerieren bzw. aufarbeiten lassen, weil sie keine diese Prozesse störenden Salze enthalten.

Selbstverständlich lassen sich auch die Metallsalze der Aminoarylsulfonsäuren nach dem erfindungsgemässen Verfahren acetylieren, jedoch werden dann, wie oben ausgeführt, die N-Acetylaminoarylsulfonsäuren nicht salzfrei erhalten, und es fallen Filtrat- und Waschflüssigkeiten an, deren Regenerierbarkeit durch die darin enthaltenen Salze erheblich eingeschränkt ist.

Die N-Acetylaminoarylsulfonsäuren und ihre Chloride sind wertvolle Zwischenprodukte bei der Herstellung von Derivaten der Aminoarylsulfonsäuren (z. B. ihrer Säurechloride und -amide oder von Sulfonen). Oft ist es nämlich erforderlich, vor ihrer jeweiligen Umsetzung die Aminogruppe durch Einführung einer Acetylgruppe zu schützen. Die Acetylgruppe wird in einer geeigneten späteren Verfahrensstufe durch Hydrolyse wieder abgespalten.

*Beispiele*

In den folgenden Beispielen bedeuten Teile jeweils Gew.-Teile, sofern nichts anderes angegeben ist. Ebenso beziehen sich %-Angaben auf das Gewicht, sofern nichts anderes angegeben ist.

*Beispiel 1:*

a) *m-Acetaminobenzolsulfonsäure (N-Acetylmetanilsäure)*

In einem mit Rührer versehenen Dreihalsglaskolben wurden unter Rühren in ein bei etwa 20° C hergestelltes Gemisch aus 645 Teilen Acetanhydrid und 1005 Teilen 99,5%iger Schwefelsäure während 1 h bei allmählich von etwa 20 auf etwa 40° C ansteigender Temperatur 866 Teile getrocknete Metanilsäure eingetragen. Zur Beendigung der Acetylierung wurde auf etwa 80° C erhitzt und bei dieser Temperatur nachgerührt unter Nachdosierung von 60 Teilen Acetanhydrid. Das Reaktionsgemisch wurde dann in 1000 Teile in einem offenen Gefäss vorgelegtes Wasser bei etwa 35° C eingerührt, wobei die N-Acetylmetanilsäure in Form eines Hydrates auskristallisierte. Man rührte bei etwa 15° C nach und saugte ab. Man erhielt 1350 Teile eines etwa 75%igen Produktes (mit anhaftender wässeriger Schwefel- und Essigsäure), das in 1355 Teile in einem offenen Glasgefäss vorgelegte 26%ige Salzsäure bei etwa 5° C eingerührt wurde. Die entstandene weisse Suspension wurde abgesaugt und mit 580 Teilen 31%iger Salzsäure gewaschen.

Man erhielt 1380 Teile eines etwa 73%igen schwefelsäurefreien N-Acetylmetanilsäure Hydrats mit einem Gehalt von etwa 100 Teilen Chlorwasserstoff. Das Produkt wurde bei von etwa 25 auf 120° C langsam steigender Temperatur unter einem Stickstoffstrom bei etwa 200 mbar in einem Trockenschrank getrocknet und anschliessend gemahlen.

Man erhielt 1020 Teile N-Acetylmetanilsäure als etwa 99%iges Pulver mit einem Schmelzpunkt von 215-220° C, was einer Ausbeute von 95% d. Th. entsprach.

Ausserdem wurden erhalten

2180 Teile einer salz- und salzsäurefreien, regenerierbaren, etwa 40%igen Filtratschwefelsäure mit einem Gehalt von 430 Teilen Essigsäure, 1820 Teile einer etwa 22%igen Filtratsalzsäure mit einem Gehalt von 100 Teilen Schwefelsäure und 35 Teilen Essigsäure.

b) *N-Acetylmetanilsäurechlorid*

In einem mit gasdichtem Rührer, Einfüll- und Abgasstutzen versehenen Glaskolben wurden 233 Teile Chlorsulfonsäure vorgelegt und unter Rühren 215 Teile der gemäss Beispiel 1a erhaltenen N-Acetylmetanilsäure während 1 h bei langsam von etwa 25 auf etwa 55° C ansteigender Temperatur mittels einer Dosierschnecke eingetragen. Danach liess man bei etwa 55° C während 4 h 238 Teile Thionylchlorid zulaufen. Nach Beendigung der Gasentwicklung (HCl, SO$_2$; das erstere wurde in üblicher Weise zunächst beim Durchleiten durch Wasser absorbiert, das letztere anschliessend mittels Natronlauge) wurde das Reaktionsgemisch in 1000 Teile Eiswasser (aus 800 Teilen Wasser und 200 Teilen Eis), in dem 30 Teile Aktivkohlepulver suspendiert waren, eingerührt. Das ausgefällte Produkt wurde abgesaugt und mit 800 Teilen Eiswasser gewaschen. Das Waschfiltrat diente zur Fällung des nächsten Ansatzes.

Man erhielt 210 Teile N-Acetylmetanilsäurechlorid als technisch feuchtes Produkt in einer Ausbeute von 83% d. Th., bezogen auf Metanilsäure.

Ausserdem wurden erhalten

1130 Teile salzfreie, regenerierbare, etwa 15%ige Schwefelsäure mit einem Gehalt von 65 Teilen Chlorwasserstoff und 17 Teilen Metanilsäure, 800 Teile Waschfiltrat mit 10 Teilen Chlorwasserstoff und 15 Teilen Schwefelsäure, 300 Teile etwa 23%ige Salzsäure (aus der Abgaswäsche) mit einem Gehalt von etwa 20 Teilen SO$_2$, 200 Teile etwa 25%ige, nahezu chloridfreie Natriumhydrogensulfitlösung (aus der Abgaswäsche).

*Beispiele 2 bis 9:*

*N-Acetylmetanilsäurechlorid ohne Zwischenisolierung von N-Acetylmetanilsäure*

*Beispiel 2:*

In dem in Beispiel 1 b verwendeten Glaskolben wurden 159 Teile Acetylchlorid vorgelegt und bei 20° C unter Rühren und Aussenkühlug 200 Teile wasserfreie Schwefelsäure zutropfen gelassen. Danach wurden 173 Teile trockene Metanilsäure bei etwa 45° C mittels einer Dosierschnecke eingetragen. Zur Beendigung der Acetylierung wurde auf etwa 95° C erhitzt.

Nach dem Abkühlen wurde die erhaltene Lösung der N-Acetylmetanilsäure ohne Zwischenisolierung derselben bei etwa 30° C mit 233 Teilen Chlorsulfonsäure verdünnt. Bei etwa 55° C wurden während etwa 4 h 417 Teile Thionylchlorid unter Rühren zulaufen gelassen. Nach Beendigung der Gasentwicklung (SO$_2$, HCl; ihre Absorption geschah, wie in Beispiel 1b beschrieben, in üblicher Weise) wurde das Reaktionsgemisch in 1500 Teile Eiswasser (aus je 750 Teilen Wasser und Eis), in dem 30 Teile Aktivkohlepulver suspendiert waren, eingerührt. Das ausgefällte Produkt wurde abgesaugt und mit 1500 Teilen Eiswasser gewaschen. Das Waschfiltrat kann zur Fällung des nächsten Ansatzes dienen.

Man erhielt 210 Teile N-Acetylmetanilsäurechlorid als technisch feuchtes Produkt in einer Ausbeute von 90% d. Th., bezogen auf Metanilsäure.

Ausserdem wurden erhalten

1763 Teile salzfreie, regenerierbare, etwa 19%ige Filtratschwefelsäure mit einem Gehalt von 127 Teilen Chlorwasserstoff, 30 Teilen Essigsäure und 17 Teilen Metanilsäure, 1546 Teile Waschfiltrat mit 15 Teilen Chlorwasserstoff und 40 Teilen Schwefelsäure, 771 Teile etwa 27%ige Salzsäure (aus der Abgaswäsche) mit einem Gehalt von 60 Teilen SO$_2$ und 30 Teilen Essigsäure, 1008 Teile etwa 21%ige, nahezu chloridfreie Natriumhydrogensulfitlösung (aus der Abgaswäsche).

*Beispiel 3:*

In dem in Beispiel 1b verwendeten Glaskolben wurden unter Rühren in ein bei etwa 20° C hergestelltes Gemisch aus 645 Teilen Acetanhydrid und 1000 Teilen wasserfreier Schwefelsäure während 1 h bei allmählich von etwa 20 auf etwa 40° C ansteigender Temperatur 865 Teile getrocknete Metanilsäure mittels einer Dosierschnecke eingetragen. Zur Beendigung der Acetylierung wurde auf etwa 85° C erhitzt und bei dieser Temperatur nachgerührt unter Nachdosierung von 60 Teilen Acetanhydrid.

Nach dem Abkühlen auf etwa 50° C wurde die erhaltene Lösung der N-Acetylmetanilsäure ohne Zwischenisolierung derselben mit 583 Teilen Chlorsulfonsäure verdünnt. Bei etwa 55° C wurden während etwa 8 h 2678 Teile Thionylchlorid unter Rühren zulaufen gelassen. Es wurde dann bei etwa 55° C noch 2 h nachgerührt. Nach Beendigung der Gasentwicklung (SO$_2$, HCl; ihre Absorption geschah, wie in Beispiel 1b beschrieben, in üblicher Weise) wurde das Reaktionsgemisch im trockenen Stickstoffstrom von Restgasen befreit und danach in 5500 Teile Eiswasser (aus je 2750 Teilen Wasser und Eis) eingerührt. Das ausgefällte Produkt wurde abgesaugt und mit 5500 Teilen Eiswasser gewaschen. Das Waschfiltrat diente zur Fällung des nächsten Ansatzes.

Man erhielt 935 Teile N-Acetylmetanilsäurechlorid als technisch feuchtes Produkt in einer Ausbeute von 80% d. Th., bezogen auf Metanilsäure.

Der zu Beginn der Fällung auftretende gummiartige, klebrige Zustand des Produkts konnte dadurch vermieden werden, dass man vor der Fällung in den 5500 Teilen Eiswasser 150 Teile Aktivkohlepulver oder 100 Teile Produkt aus einem früheren Ansatz suspendierte.

Ausserdem wurden erhalten

6700 Teile salzfreie, regenerierbare, etwa 19%ige Filtratschwefelsäure mit einem Gehalt von 430 Teilen Chlorwasserstoff, 430 Teilen Essigsäure und 170 Teilen Metanilsäure, 5000 Teile Waschfiltrat mit 80 Teilen Chlorwasserstoff und 210 Teilen Schwefelsäure, 4700 Teile etwa 25%ige Salzsäure (aus der Abgaswäsche) mit einem Gehalt von 205 Teilen $SO_2$ und 140 Teilen Essigsäure, 8200 Teile etwa 22%ige, nahezu chloridfreie (Kochsalzgehalt <1%) Natriumhydrogensulfitlösung (aus der Abgaswäsche) mit einem geringen Gehalt (<10 Teile) Essigsäure.

*Beispiel 4:*

Man verfuhr wie in Beispiel 3 mit dem Unterschied, dass man 2975 Teile Thionylchlorid während 10 h zulaufen liess.

Man erhielt ebenfalls 935 Teile N-Acetylmetanilsäurechlorid, was einer unveränderten Ausbeute von 80% d. Th. entsprach.

*Beispiel 5:*

Man verfuhr wie in Beispiel 3 mit dem Unterschied, dass man unmittelbar vor der Zugabe des Thionylchlorids 25 Teile Dimethylformamid in das Reaktionsgemisch zulaufen liess.

Man erhielt 970 Teile N-Acetylmetanilsäurechlorid, was einer Ausbeute von 83% d. Th. entsprach. In der Salzsäure aus der Abgaswäsche fanden sich 240 Teile Essigsäure.

*Beispiel 6:*

Man verfuhr wie in Beispiel 4 mit dem Unterschied, dass zum Gemisch aus 645 Teilen Acetanhydrid und 1000 Teilen wasserfreier Schwefelsäure 25 Teile Dimethylforamid hinzugefügt wurden.

Man erhielt 1040 Teile N-Acetylmetanilsäurechlorid, was einer Ausbeute von 89% d. Th. entsprach.

*Beispiel 7:*

Man verfuhr wie in Beispiel 4 mit dem Unterschied, dass den 1000 Teilen wasserfreier Schwefelsäure 5 Teile Wasser hinzugefügt wurden.

Man erhielt 1017 Teile N-Acetylmetanilsäurechlorid, was einer Ausbeute von 87% d. Th. entsprach.

*Beispiel 8:*

Man verfuhr wie in Beispiel 7 mit dem Unterschied, dass 10 Teile Wasser zugegeben wurden.

Man erhielt ebenfalls 1017 Teile N-Acetylmetanilsäurechlorid.

*Beispiel 9:*

Man verfuhr sowohl wie in Beispiel 7 als auch wie in Beispiel 5. Man erhielt 1040 Teile N-Acetylmetanilsäurechlorid, was einer Ausbeute von 89% d. Th. entsprach.

*Beispiel 10:*

a) *p-Acetaminobenzolsulfonsäure (N-Acetylsulfanilsäure)*

In dem in Beispiel 1a verwendeten Glaskolben wurde ein bei etwa 15° C hergestelltes Gemisch aus 205 Teilen Acetanhydrid, 5 Teilen Dimethylformamid und 300 Teilen wasserfreier Schwefelsäure vorgelegt. Bei allmählich von etwa 15 auf etwa 30° C ansteigender Temperatur wurden unter Rühren 173 Teile getrocknete Sulfanilsäure während etwa 1 h eingetragen. Zur Beendigung der Acetylierung wurde auf etwa 65° C erhitzt und bei dieser Temperatur nachgerührt. Das Reaktionsgemisch wurde dann in 300 Teile Wasser von etwa 35° C eingerührt, wobei die N-Acetylsulfanilsäure in Form eines Hydrates auskristallisierte. Die Kristallisation wurde durch Nachrühren bei etwa 15° C vervollständigt. Man erhielt nach dem Absaugen 293 Teile eines etwa 60%igen Produkts, das in 500 Teile 26%ige Salzsäure bei etwa 5° C eingerührt wurde. Die entstandene weisse Suspension wurde abgesaugt und mit 250 Teilen kalter 26%iger Salzsäure gewaschen.

Man erhielt 304 Teile eines schwefelsäurefreien, salzsäurehaltigen N-Acetylsulfanilsäure-Hydrats. Das Produkt wurde bei von etwa 25 auf etwa 90° C langsam steigender Temperatur unter einem Stickstoffstrom von etwa 200 mbar in einem Trockenschrank getrocknet.

Man erhielt 193 Teile N-Acetylsulfanilsäure als ein 99%iges Pulver mit einem Schmelzpunkt von 243-246° C, was einer Ausbeute von 90% d. Th. entsprach.

Ausserdem wurden erhalten

733 Teile einer salz- und salzsäurefreien, regenerierbaren, etwa 35%igen Filtratschwefelsäure mit einem Gehalt von 144 Teilen Essigsäure, 712 Teile einer etwa 21%igen Filtratsalzsäure mit einem Gehalt an 25 Teilen Schwefelsäure und 10 Teilen Essigsäure.

b) *p-Acetaminobenzolsulfochlorid (N-Acetylsulfanilsäurechlorid)*

In dem in Beispiel 1b verwendeten Glaskolben wurden 117 Teile Chlorsulfonsäure vorgelegt und unter Rühren 114 Teile der gemäss Beispiel 10a erhaltenen N-Acetylsulfanilsäure während 1 h bei langsam von etwa 25 auf etwa 45° C ansteigender Temperatur eingetragen. Nach vollständiger Lösung wurden 2,50 Teile Dimethylformamid zugegeben. Dann liess man bei etwa 50-55° C während 4 h 119 Teile Thionylchlorid zulaufen. Man rührte noch 2 h bei 50-55° C nach. Nach Beendigung der Gasentwicklung (HCl, $SO_2$; ihre Absorption geschah wie in Beispiel 1b beschrieben, in üblicher Weise) wurde das Reaktionsgemisch im trockenen Stickstoffstrom von Restgasen befreit und danach in 500 Teile Eiswasser (aus je 250 Teilen Wasser und Eis) eingerührt. Das ausgefällte Produkt wurde abgesaugt und mit 500 Teilen Eiswasser gewaschen. Man erhielt 119 Teile p-Acetaminobenzolsulfochlorid als technisch feuchtes Produkt in einer Ausbeute von 86% d. Th. auf Sulfanilsäure.

Ausserdem wurden erhalten

640 Teile salzfreie, regenerierbare, etwa 22%ige Schwefelsäure mit einem Gehalt von 43 Teilen Chlorwasserstoff, 480 Teile Waschfiltrat mit 6 Teilen Chlorwasserstoff und 13 Teilen Schwefelsäure, 140 Teile etwa 20%ige Salzsäure (aus der Abgaswäsche) mit einem Gehalt von 13 Teilen $SO_2$, 95 Teile etwa 20%ige Natriumhydrogensulfitlösung (aus der Abgaswäsche).

*Beispiel 11:*

*o-Acetaminobenzolsulfonsäure (N-Acetylorthanilsäure)*

Man verfuhr wie in Beispiel 10 a mit dem Unterschied, dass anstelle von 173 Teilen Sulfanilsäure die gleiche Menge getrocknete Orthanilsäure eingesetzt wurde.

Man erhielt 133 Teile N-Acetylorthanilsäure als ein etwa 97%iges Pulver mit einem Schmelzpunkt von 228-230° C. Die Ausbeute betrug 62% d. Th.

Ausserdem wurden erhalten

723 Teile einer salz- und salzsäurefreien, regenerierbaren, etwa 36%igen Filtratschwefelsäure mit einem Gehalt von 155 Teilen Essigsäure, 793 Teile einer etwa 22%igen Filtratsalzsäure mit einem Gehalt an 36 Teilen Schwefelsäure und 48 Teilen Essigsäure.

*Beispiel 12:*

a) *2-Acetaminophthalin-8-sulfonsäure*

In dem in Beispiel 1a verwendeten Glaskolben wurde ein bei etwa 15° C hergestelltes Gemisch aus 1025 Teilen Acetanhydrid und 1500 Teilen wasserfreier Schwefelsäure vorgelegt. Bei allmählich von etwa 20 auf etwa 45° C ansteigender Temperatur wurden unter Rühren 1117 Teile trockene 2-Aminonaphthalin-8-sulfonsäure während etwa 1 h eingetragen. Zur Beendigung der Acetylierung wurde auf etwa 80° C erhitzt und bei dieser Temperatur nachgeführt. Das Reaktionsgemisch wurde dann in 2500 Teile Wasser bei etwa 35° C eingerührt, wobei die 2-Acetaminonaphthalin-8-sulfonsäure auskristallisierte. Die Kristallisation wurde durch Nachrühren bei etwa 15° C vervollständigt. Nach dem Absaugen erhielt man 1800 Teile eines etwa 70%igen Produktes. Dieses wurde in ein Gemisch aus 1750 Teilen 31%iger Salzsäure und 300 Teilen Eis eingerührt. Die entstandene hellgraue Suspension wurde bei etwa 5° C abgesaugt und mit 580 Teilen 31%iger Salzsäure gewaschen.

Man erhielt 1670 Teile eines schwefelsäurefreien, salzsäurehaltigen, 75%igen 2-Acetaminonaphthalin-8-sulfonsäure-Hydrats. Das Produkt wurde bei von etwa 25 auf etwa 120° C langsam steigender Temperatur unter einem Stickstoffstrom von etwa 200 mbar in einem Trockenschrank getrocknet.

Man erhielt 1260 Teile 2-Acetaminonaphtalin-8-sulfonsäure als ein 97%iges Pulver, was einer Ausbeute von 95% d. Th. entsprach, bezogen auf 2-Aminonaphthalin-8-sulfonsäure.

Ausserdem wurden erhalten

3800 Teile einer salz- und salzsäurefreien, regenerierbaren, etwa 30%igen Filtratschwefelsäure mit einem Gehalt von 780 Teilen Essigsäure, 2250 Teile einer etwa 22%igen Filtratsalzsäure mit einem Gehalt von 185 Teilen Schwefelsäure.

b) *2-Acetaminonaphthalin-8-sulfonsäurechlorid*

In dem in Beispiel 1b verwendeten Glaskolben wurden 466 Teile Chlorsulfonsäure vorgelegt und unter Rühren 265 Teile der gemäss Beispiel 12a erhaltenen 2-Acetaminonaphthalin-8-sulfonsäure während 2 h bei langsam von etwa 25 auf etwa 50° C ansteigender Temperatur eingetragen. Nach Beendigung der Gasentwicklung liess man bei etwa 45-50° C während 5 h 238 Teile Thionylchlorid zulaufen. Nach Beendigung der Gasentwicklung ($SO_2$, HCl; ihre Absorption geschah, wie in Beispiel 1b beschrieben, in üblicher Weise) wurde das Reaktionsgemisch in 2000 Teile Eiswasser (aus je 1000 Teilen Wasser und Eis), in dem 30 Teile Aktivkohlepulver suspendiert waren, eingerührt. Das ausgefällte Produkt wurde abgesaugt und mit 1200 Teilen Eiswasser gewaschen. Das Waschfiltrat kann zur Fällung des nächsten Ansatzes dienen.

Man erhielt 283 Teile 2-Acetaminonaphthalin-8-sulfonsäurechlorid als technisch feuchtes Produkt in einer Ausbeute von 95% d. Th., bezogen auf 2-Aminonaphthalin-8-sulfonsäure.

Ausserdem wurden erhalten

1660 Teile salzfreie, regenerierbare, etwa 14%ige Schwefelsäure mit einem Gehalt von 75 Teilen Chlorwasserstoff, 1150 Teile Waschfiltrat mit 40 Teilen Chlorwasserstoff und 110 Teilen Schwefelsäure, 300 Teile etwa 30%ige Salzsäure (aus der Abgaswäsche) mit einem Gehalt von 10 Teilen $SO_2$, 375 Teile etwa 24%ige, nahezu chloridfreie Natriumhydrogensulfitlösung (aus der Abgaswäsche).

*Beispiel 13:*

In dem in Beispiel 1b verwendeten Glaskolben wurden unter Rühren in ein bei etwa 15° C hergestelltes Gemisch aus 205 Teilen Acetanhydrid, 5 Teilen Dimethylformamid und 300 Teilen wasserfreier Schwefelsäure während 1 h bei allmählich von etwa 20 auf etwa 40° C ansteigenger Temperatur 223 Teile getrocknete 2-Aminonaphthalin-8-sulfonsäure eingetragen. Zur Beendigung der Acetylierung wurde auf etwa 80° C erhitzt und 3 h bei dieser Temperatur nachgerührt.

Nach dem Abkühlen auf etwa 50° C wurde die erhaltenen Lösung der 2-Acetaminonaphthalin-8-sulfonsäure ohne Zwischenisolierung derselben mit 233 Teilen Chlorsulfonsäure verdünnt und bei etwa 50° C bis zum Ende der Gasentwicklung nachgerührt. Bei etwa 50° C liess man während etwa 8 h 833 Teile Thionylchlorid unter Rühren zutropfen. Es wurde dann bei etwa 50° C noch 2 h nachgerührt. Nach Beendigung der Gasentwicklung ($SO_2$, HCl; ihre Absorption geschah, wie in Beispiel 1b beschrieben, in üblicher Weise) wurde das Reaktionsgemisch im trockenen Stickstoffstrom von Restgasen befreit und danach in 2000 Teile Eiswasser (aus je 1000 Teilen Wasser und Eis), dem noch 30 Teile Aktivkohle-Pulver zugesetzt waren, eingerührt. Das ausgefällte Produkt wurde abgesaugt und mit 2000 Teilen Eiswasser gewaschen.

Man erhielt 255 Teile N-Acetaminonaphtalin-8-sulfonsäure-chlorid als technisch feuchtes Produkt in einer Ausbeute von 90% d. Th., bezogen auf 2-Aminonaphtalin-8-sulfonsäure.

Ausserdem wurden erhalten

2000 Teile salzfreie, regenerierbare, etwa 15%ige Filtratschwefelsäure mit einem Gehalt von 145 Teilen Chlorwasserstoff und etwa 145 Teilen Essigsäure, 2260 Teile Waschfiltrat mit 61 Teilen Chlorwasserstoff und 170 Teilen Schwefelsäure, 890 Teile etwa 35%ige Salzsäure (aus der Abgaswäsche) mit einem Gehalt von 33 Teilen $SO_2$ und 5 Teilen Essigsäure, 5100 Teile etwa 11%ige, nahezu chloridfreie (Kochsalzgehalt <3%) Natriumhydrogensulfitlösung (aus der Abgaswäsche) mit 4 Teilen Essigsäure.

### Beispiel 14:

#### a) 2-Acetaminonaphthalin-6-sulfonsäure

In dem in Beispiel 1a verwendeten Glaskolben wurde ein bei etwa 15° C hergestelltes Gemisch aus 1025 Teilen Acetanhydrid und 1515 Teilen 99,0%iger Schwefelsäure vorgelegt. Bei allmählich von etwa 20 auf etwa 75° C ansteigender Temperatur wurden unter Rühren 1117 Teile trockene 2-Aminonaphthalin-6-sulfonsäure während etwa 2 h eingetragen. Danach wurde bei etwa 85° C nachgerührt. 3 h nach der Zugabe der 2-Aminonaphthalin-6-sulfonsäure wurden weitere 255 Teile Acetanhydrid zulaufen gelassen. Zur Beendigung der Acetylierung wurde noch 2 h bei etwa 85° C nachgerührt. Das Reaktionsgemisch wurde dann in 3000 Teile Wasser bei etwa 35° C eingerührt, wobei die 2-Acetaminonaphthalin-6-sulfonsäure auskristallisierte. Die Kristallisation wurde durch Nachrühren bei etwa 25° C vervollständig. Nach dem Absaugen erhielt man 3000 Teile eines etwa 35%igen Produktes. Dieses wurde in ein Gemisch aus 3500 Teilen 31%iger Salzsäure und 1000 Teilen Eis eingerührt. Die entstandene hellgraue Suspension wurde bei etwa 25° C abgesaugt und mit 2750 Teilen 20%iger Salzsäure gewaschen.

Man erhielt etwa 2600 Teile eines schwefelsäurefreien, salzsäurehaltigen 40%igen 2-Acetaminonaphthalin-6-sulfonsäure-Hydrats. Das Produkt wurde bei von etwa 25 auf etwa 90° C langsam steigender Temperatur unter einem Stickstoffstrom von etwa 200 mbar in einem Trockenschrank getrocknet.

Man erhielt 1140 Teile 2-Acetaminonaphthalin-6-sulfonsäure als etwa 95%igen Pulver, was einer Ausbeute von 80% d. Th. entsprach, bezogen auf 2-Aminonaphthalin-6-sulfonsäure.

Ausserdem wurden erhalten

3400 Teile einer salz- und salzsäurefreien, regenerierbaren, etwa 25%igen Filtratschwefelsäure mit einem Gehalt von 670 Teilen Essigsäure, 7300 Teile einer etwa 20%igen Filtratsalzsäure mit einem Gehalt von 440 Teilen Schwefelsäure und 380 Teilen Essigsäure.

#### b) 2-Acetaminonaphthalin-6-sulfonsäurechlorid

In dem in Beispiel 1b verwendeten Glaskolben wurde ein Gemisch aus 583 Teilen Chlorsulfonsäure, 15 Teilen Essigsäureanhydrid und 5 Teilen Dimethylformamid vorgelegt und unter Rühren 265 Teile der nach Beispiel 14a erhaltenen 2-Acetaminonaphthalin-6-sulfonsäure während 1 h bei langsam von etwa 20 auf etwa 35° C ansteigender Temperatur eingetragen. Danach liess man bei etwa 55° C während 5 h 238 Teile Thionylchlorid zulaufen. Es wurde noch etwa 2 h bei etwa 55° C nachgerührt. Nach Beendigung der Gasentwicklung ($SO_2$, HCl; ihre Absorption geschah, wie in Beispiel 1b beschrieben, in üblicher Weise) wurde durch das Reaktionsgemisch Stickstoff geblasen und der Kolbeninhalt während 1 h in 2200 Teile Eiswasser (aus 500 Teilen Wasser und 1700 Teilen Eis), in dem 30 Teile Aktivkohlepulver suspendiert waren, eingerührt. Das ausgefällt Produkt wurde abgesaugt und mit 1500 Teilen Eiswasser gewaschen. Das Waschfiltrat kann zur Fällung des nächsten Ansatzes dienen.

Man erhielt 283 Teile 2-Acetaminonaphthalin-6-sulfonsäurechlorid als technisch feuchtes Produkt in einer Ausbeute von 80% d. Th., bezogen auf 2-Aminonaphthalin-6-sulfonsäure.

Ausserdem wurden erhalten.

2330 Teile salzfreie, regenerierbare, etwa 15%ige Abfallschwefelsäure mit einem Gehalt von 135 Teilen Chlorwasserstoff sowie geringen Gehalten an essigsäure und Dimethylformamid, 1620 Teile Waschfiltrat mit 40 Teilen Chlorwasserstoff und 100 Teilen Schwefelsäure, 300 Teile etwa 23%ige Salzsäure (aus der Abgaswäsche) mit einem Gehalt von 20 Teilen $SO_2$, 700 Teile etwa 12%ige, nahezu chloridfreie (Kochsalzgehalt <1%) Natriumhydrogensulfitlösung (aus der Abgaswäsche).

### Beispiel 15:

In dem in Beispiel 1b verwendeten Glaskolben wurden unter Rühren in ein bei etwa 15° C hergestelltes Gemisch aus 205 Teilen Acetanhydrid, 5 Teilen Dimethylformamid und 300 Teilen wasserfreier Schwefelsäure während 2 h bei allmählich von etwa 20 auf etwa 70-85° C ansteigender Temperatur 223 Teile getrocknete 2-Aminonaphthalin-6-sulfonsäure eingetragen. Zur Beendigung der Acetylierung wurde bei etwa 85° C 3 h nachgerührt. Dann wurden nochmals 51 Teile Acetanhydrid bei etwa 50° C zugesetzte und das Nachrühren bei etwa 85° C noch 2 h fortgesetzt.

Nach dem Abkühlen auf etwa 50° C wurde die erhaltene Lösung der 2-Acetaminonaphthalin-6-sulfonsäure ohne Zwischenisolierung derselben mit 233 Teilen Chlorsulfonsäure verdünnt und bei etwa 50° C bis zum Ende der Gasentwicklung nachgerührt. Bei etwa 50° C liess man während etwa 8 h 833 Teile Thionylchlorid unter Rühren zutropfen. Es wurde dann bei etwa 50° C noch 2 h nachgerührt. Nach Beendigung der Gasentwicklung ($SO_2$, HCl; ihre Absorption geschah, wie in Beispiel 1b beschrieben, in üblicher Weise) wurde das Reaktionsgemisch im trockenen Stickstoffstrom von Restgasen befreit und danach in 2000 Teile Eiswasser (aus je 1000 Teilen Wasser und Eis), dem noch 30 Teile Aktivkohlepulver zugesetzt waren, eingerührt. Das ausgefällte Produkt wurde abgesaugt und mit 2000 Teilen Eiswasser gewaschen.

Man erhielt 251 Teile 2-Acetaminonaphthalin-6-sulfonsäurechlorid als technisch feuchtes Produkt in einer Ausbeute von 89% d. Th., bezogen auf 2-Aminonaphthalin-6-sulfonsäure.

Ausserdem wurden erhalten

2370 Teile salzfreie, regenerierbare, etwa 15%ige Filtratschwefelsäure mit einem Gehalt von 117 Teilen Chlorwasserstoff und 92 Teilen Essigsäure, 2110 Teile Waschfiltrat mit 31 Teilen Chlorwasserstoff und 83 Teilen Schwefelsäure, 887 Teile etwa 35%ige Salzsäure (aus der Abgaswäsche) mit einem Gehalt von 36 Teilen $SO_2$ und 81 Teilen Essigsäure, 5260 Teile etwa 11%ige, nahezu chloridfreie (Kochsalzgehalt <3%) Natriumhydrogensulfitlösung (aus der Abgaswäsche) mit 41 Teilen Essigsäure.

*Beispiel 16* (Vergleichsbespiel):

a) In dem in Beispiel 1 a verwendeten Glaskolben wurden 173 Teile Metanilsäure (als wasserfeuchtes Produkt) in 300 Teilen Wasser durch Zugabe der stöchiometrischen Menge Natronlauge zum Natriumsalz neutralisiert. Anschliessend liess man bei etwa 80°C 141 Teile Acetanhydrid zulaufen. Zur Beendigung der Acetylierung wurde bei etwa 80°C nachgerührt, wobei der pH-Wert von etwa 7 auf etwa 2 absank. Danach wurden Wasser und Essigsäure (72 Teile) abdestilliert. Nach dem Trocknen im Trockenschrank bei 120°C und etwa 200 mbar wurden 237 Teile N-acetylmetanilsaures Natrium als 97%iges Produkt erhalten.

b) In dem in Beispiel 1b verwendeten Glaskolben wurden 525 Teile Chlorsulfonsäure vorgelegt und unter Rühren 237 Teile des gemäss Beispiel 16a erhaltenen N-acetylmetanilsauren Natriums während etwa 1 h bei langsam von etwa 25 auf etwa 55°C ansteigender Temperatur eingetragen. Danach liess man bei etwa 55°C während 5 h 238 Teile Thionylchlorid unter Rühren zulaufen. Nach Beendigung der Gasentwicklung ($SO_2$, HCl; ihre Absorption geschah, wie in Beispiel 1b beschrieben, in üblicher Weise) wurde das Reaktionsgemisch in 1700 Teile Eiswasser (aus 1200 Teilen Wasser und 500 Teilen Eis), in dem 30 Teile Aktivkohlepulver suspendiert waren, eingerührt. Das ausgefällte Produkt wurde abgesaugt und mit 1100 Teilen Eiswasser gewaschen. Das Waschfiltrat kann zur Fällung des nächsten Ansatzes dienen.

Man erhielt 210 Teile N-Acetylmetanilsäurechlorid als technisch feuchtes Produkt in einer Ausbeute von 90% d.Th., bezogen auf Metanilsäure.

Ausserdem wurden erhalten

2100 Teile salzhaltige (120 Teile $NaHSO_4$), etwa 20%ige Abfallschwefelsäure, deren Regeneration infolge des Salzgehaltes erheblich gestört ist, und die ferner 190 Teile Chlorwasserstoff und 17 Teile Metanilsäure enthält,

1000 Teile Waschfiltrat mit 36 Teilen Chlorwasserstoff und 40 Teilen Schwefelsäure,

300 Teile etwa 27%ige Salzsäure (aus der Abgaswäsche) mit einem Gehalt von 20 Teilen $SO_2$,

400 Teile einer etwa 18%igen Essigsäure (bei Beispiel 16a),

250 Teile etwa 27%ige Natriumhydrogensulfitlösung.

*Beispiel 17* (Vergleichsbeispiel):

Man verfuhr wie in Beispiel 16a mit dem Unterschied, dass die Zugabe der für die Neutralisation der Metanilsäure erforderlichen stöchiometrischen Menge Natronlauge unterblieb. Die Metanilsäure wurde nicht acetyliert.

## Patentansprüche

1. Verfahren zur Herstellung von N-Acetylaminoarylsulfonsäuren durch Acetylierung von Aminoarylsulfonsäuren mit Essigsäureanhydrid oder Acetylchorid, dadurch gekennzeichnet, dass man die Acetylierung in Schwefelsäure als Lösungsmittel vornimmt.

2. Verfahren nach Anspruch 1, dadurch gekennzeichnet, dass man die Acetylierung in Gegenwart geringer Mengen von Wasser und/oder Dimethylformamid und/oder N-Methylpyrrolidon vornimmt.

3. Verfahren nach einem der beiden Ansprüche 1 oder 2, dadurch gekennzeichnet, dass man die Acetylierung in Gegenwart von bis zu 2 Gew.-%, vorzugsweise bis zu 1 Gew.-%, Wasser oder in Gegenwart von bis zu 3 Gew.-%, vorzugsweise bis zu 2,5 Gew.-% Dimethylformamid oder N-Methylpyrrolidon, jeweils bezogen auf die Schwefelsäure, einsetzt.

4. Verfahren nach Ansprüchen 1 bis 3, dadurch gekennzeichnet, dass 2 bis 3 mol Schwefelsäure je Mol Aminoarylsulfonsäure zusammen mit dem Acetylierungsmittel vorgelegt werden.

5. N-Acetylmetanilsäure in fester, freier und wasserfreier Form.

6. Verwendung der N-Acetylmetanilsäure gemäss Anspruch 5 in fester, getrockneter Form oder in Form ihrer Lösungen in Schwefelsäure zur Herstellung ihres Säurechlorids.

## Claims

1. A process for the preparation of N-acetylaminoarylsulfonic acids by acetylation of aminoarylsulfonic acids with acetic anhydride or acetyl chloride, which comprises carrying out the acetylation in sulfuric acid as the solvent.

2. The process as claimed in Claim 1, wherein the acetylation is carried out in the presence of small amounts of water and/or dimethylformamide and/or N-methylpyrrolidone.

3. The process as claimed in Claim 1 or 2, wherein the acetylation is set up in the presence of up to 2% by weight, preferably up to 1% by weight, of water or in the presence of up to 3% by weight, preferably up to 2.5% by weight, of dimethylformamide or N-methylpyrrolidone, in each case relative to the sulfuric acid.

4. The process as claimed in any of Claims 1 to 3, wherein 2 to 3 mol of sulfuric acid per mole of aminoarylsulfonic acid are initially introduced together with the acetylating agent.

5. N-Acetylmetanilic acid in solid, free and anhydrous form.

7. The use of the N-acetylmetanilic acid as claimed in Claim 5, in the form of a dry solid or in the form of its solution in sulfuric acid for the preparation of its acid chloride.

## Revendications

1. Procédé pour la préparation d'acides N-acétylaminoarylsulfoniques par acétylation d'acides aminoarylsulfoniques avec de l'anhydride acétique ou du chlorure d'acétyle, caractérisé en ce qu'on procède à l'acétylation dans de l'acide sulfurique en tant que solvant.

2. Procédé selon la revendication 1, caractérisé en ce qu'on procède à l'acétylation en présence de faibles quantités d'eau et/ou de diméthylformamide et/ou de N-méthylpyrrolidone.

3. Procédé selon l'une des revendications 1 ou 2, caractérisé en ce qu'on procède à l'acétylation en présence d'une quantité d'eau allant jusqu'à 2% en poids, de préférence jusqu'à 1% en poids, ou en présence d'une quantité de diméthylformamide ou de N-méthylpyrrolidone pouvant aller jusqu'à 3% en poids, de préférence jusqu'à 2,5% en poids, dans chaque cas par rapport à l'acide sulfurique.

4. Procédé selon l'une des revendications 1 à 3, caractérisé en ce qu'on utilise 2 à 3 mol d'acide sulfurique par mole d'acide aminoarylsulfonique, en même temps que l'agent d'acétylation.

5. Acide N-acétylmétanilique, sous forme solide, libre et anhydre.

6. Utilisation de l'acide N-acétylmétanilique selon la revendication 5, sous forme solide séchée ou sous la forme de ses solutions dans de l'acide sulfurique, pour la préparation de son chlorure d'acide.